# FASCICULE DE BREVET EUROPEEN

(11) **EP 1 466 587 B1**
(45) Date de publication et mention de la délivrance du brevet: **07.11.2012**
(21) Numéro de dépôt: 04290588.5
(22) Date de dépôt: 04.03.2004
(51) Int. Cl.: A61K 8/06, A61K 8/34, A61K 8/37, A61K 8/39, A61K 8/81, A61Q 1/00, A61Q 5/00, A61Q 19/00

(54) **Composition sous forme d'une émulsion huile-dans-eau contenant un polymère amphiphile, et ses utilisations notamment cosmétiques**
Zusammensetzung in Form einer Öl-in-Wasser Emulsion und deren kosmetische Verwendungen
Composition in the form of an oil-in-water emulsion and its cosmetic uses

(30) Priorité: 11.04.2003 FR 0304578
(43) Date de publication de la demande: 13.10.2004
(73) Titulaire: L'Oréal, 75008 Paris (FR)
(72) Inventeur: Aubrun-Sonneville, Odile, 92160 Antony (FR); L'Alloret, Florence, 75013 Paris (FR)
(74) Mandataire: Duvert, Sandra

(56) Documents cités:
- EP-A- 1 069 142
- WO-A-02/43686
- WO-A-99/56727
- US-B1- 6 426 062

## Description

La présente demande se rapporte à une composition sous forme d'émulsion huile-dans-eau contenant au moins un polymère amphiphile particulier et un émulsionnant lipophile, et à l'utilisation de la dite composition, notamment pour le soin, le démaquillage et/ou le nettoyage de la peau du corps ou du visage, des cheveux, des lèvres et/ou des yeux.

Pour diverses raisons liées en particulier à un meilleur confort d'utilisation (douceur, émollience et autres), les compositions cosmétiques actuelles se présentent le plus souvent sous la forme d'une émulsion du type huile-dans-eau (H/E) constituée d'une phase continue dispersante aqueuse et d'une phase discontinue dispersée huileuse ou d'une émulsion du type eau-dans-huile (E/H) constituée d'une phase continue dispersante huileuse et d'une phase discontinue dispersée aqueuse. Les émulsions H/E sont les plus demandées dans le domaine cosmétique du fait qu'elles comportent comme phase externe, une phase aqueuse, ce qui leur confère, lors de l'application sur la peau, un toucher plus frais, moins gras et plus léger que les émulsions E/H.

Les émulsions H/E classiques sont en général stabilisées par des molécules amphiphiles de faible masse molaire (< 5 000 g/mole) tels que les tensioactifs émulsionnants de type alkylglycérolé ou alkylpolyoxyéthyléné. Toutefois, ces tensioactifs présentent l'inconvénient d'induire un toucher cireux et lourd.

Par ailleurs, il a été envisagé de remplacer les tensioactifs par des polymères amphiphiles, comportant dans leur chaîne une partie hydrophile et une partie hydrophobe constituée d'une chaîne grasse, tels que les copolymères d'alkyl-C₁₀-C₃₀-acrylate et d'acide acrylique ou méthacrylique, comme les produits commercialisés sous la dénomination PEMULEN par la société Noveon. Cependant, ces polymères épaississent les compositions et présentent donc l'inconvénient de ne pas permettre l'obtention de compositions fluides. Par ailleurs, les émulsions stabilisées par ce type de polymère comprennent des gouttes d'huile de taille relativement élevée, ce qui ne permet pas d'obtenir une stabilité acceptable pour de faibles niveaux de viscosité, l'instabilité se traduisant notamment par un crémage rapide, c'est-à-dire une remontée d'huile à la partie supérieure de l'émulsion, ce qui se traduit par une séparation de phase avec une phase blanche en haut du flacon et une phase limpide aqueuse en bas du flacon.

En outre, il est connu par le document EP-A-1,069,142 d'utiliser des polymères amphiphiles de monomères à insaturation α,β-éthylénique et de monomères hydrophobes, obtenus par polymérisation radicalaire d'un macromonomère contenant un bloc hydrophile de type polyoxyalkyléné et un bloc hydrophobe comprenant de 1 à 30 atomes de carbone, ces polymères pouvant être utilisés en comme épaississant, émulsionnant, dispersant et agent de suspension, notamment dans le domaine cosmétique. Toutefois, les polymères exemplifiés dans ce document ne permettent pas d'obtenir des émulsions H/E ayant des propriétés cosmétiques très agréables pour l'utilisateur tout en étant très stables et de réalisation aisée.

Il subsiste donc le besoin de réaliser des émulsions H/E à base de polymères amphiphiles, surmontant les difficultés de l'art antérieur, c'est-à-dire étant particulièrement intéressantes comme toucher cosmétique tout en étant de réalisation aisée et tout en présentant une bonne stabilité.

La demanderesse a découvert de façon inattendue que l'association de polymères amphiphiles particuliers et d'émulsionnants lipophiles particuliers permettait de réaliser de telles émulsions. On peut ainsi obtenir des émulsions huile-dans-eau, agréables à utiliser, qui restent stables dans le temps à température ambiante ou à des températures plus élevées.

Ainsi, la présente invention concerne une composition pour application topique, sous forme d'émulsion huile-dans-eau comprenant une phase huileuse dispersée dans une phase aqueuse, caractérisée par le fait qu'elle contient au moins un émulsionnant lipophile et au moins un polymère amphiphile comprenant de (A) 80 à 99 % en moles de motif acide 2-acrylamido 2-méthylpropane sulfonique de formule (I) : 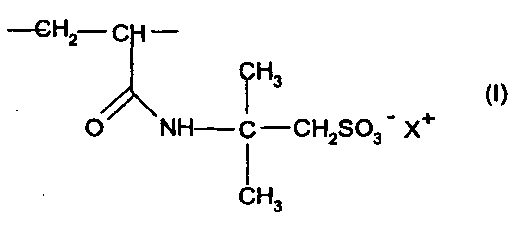 dans laquelle X⁺ est un proton, un cation de métal alcalin, un cation alcalino-terreux ou l'ion ammonium ;
et (B) 1 à 20 % en moles de motif de formule (II) suivante : 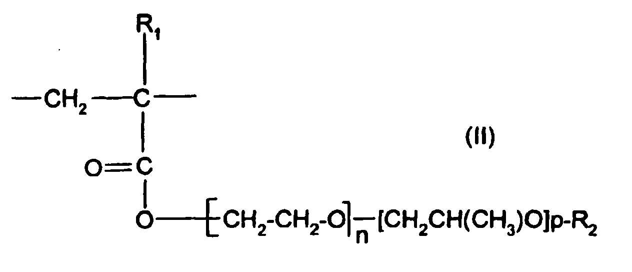 dans laquelle n et p, indépendamment l'un de l'autre, désignent un nombre entier et varie de 0 à 30, de préférence de 1 à 25 et plus préférentiellement de 3 à 20, sous réserve que n + p soit inférieur ou égal à 30, de préférence inférieur à 25 et encore mieux inférieur à 20 ; R₁ désigne un atome d'hydrogène, un radical alkyle linéaire ou ramifié comportant de 1 à 6 atomes de carbone et de préférence un radical méthyle, et R₂ désigne un radical alkyle linéaire ou ramifié comportant de 6 à 15 atomes de carbone, de préférence de 8 à 15 atomes de carbone.

La proportion en moles de monomère hydrophobe de formule (II) correspond au taux de greffage des polymères.

On entend ici par « application topique », une application externe sur les matières kératiniques, que sont notamment la peau, le cuir chevelu, les cils, les sourcils, les ongles, les muqueuses et les cheveux.

La composition selon l'invention étant destinée à une application topique, elle contient un milieu physiologiquement acceptable, c'est-à-dire compatible avec la peau, les muqueuses, les cheveux et le cuir chevelu.

La composition de l'invention a une texture homogène et agréable à l'application. En outre, elle est bien stable à la conservation. Par "stable", on entend une composition qui, après deux mois de stockage ou plus à toutes températures comprises entre 4°C et 50°C, ne présente ni changement macroscopique de couleur, d'odeur ou de viscosité, ni variation de pH, ni changement d'apparence microscopique.

La composition de l'invention a une viscosité qui peut varier largement selon le but final recherché. Ainsi, sa viscosité peut aller par exemple de 0,005 Pa.s à 1 Pa.s à une température de 25°C pour une vitesse de cisaillement de 200 s⁻¹.

### Polymères

Le polymère amphiphile utilisé dans la composition de l'invention est hydrosoluble ou hydrodispersible. On entend par "polymère hydrosoluble ou hydrodispersible", un polymère qui, introduit dans de l'eau à une concentration égale à 1 % en poids, conduit à une solution macroscopiquement homogène dont la transmittance de la lumière, à une longueur d'onde égale à 500 nm, à travers un échantillon de 1 cm d'épaisseur, est d'au moins 10 %, ce qui correspond à une absorbance [abs = -log (transmittance)] inférieure à 1,5.

De manière caractéristique, les solutions aqueuses des polymères utilisés selon l'invention présentent, à une concentration de 1 % en poids en polymère, une viscosité inférieure à 1 Pa.s pour une vitesse de cisaillement égale à 1 s⁻¹, la viscosité étant mesurée à 25°C à l'aide d'un rhéomètre à contrainte imposée de type RS 150 (Haake).

Les polymères amphiphiles conformes à l'invention ont généralement un poids moléculaire moyen en nombre allant de 50 000 à 10 000 000, de préférence de 100 000 à 8 000 000 et plus préférentiellement de 100 000 à 7 000 000.

Les polymères utilisés selon l'invention sont tels que définis ci-dessus. Ce sont des polymères non réticulés, comprenant de (A) 80 à 99 % en moles et de préférence de 85 à 99 % en moles de motif acide 2-acrylamido 2-méthylpropane sulfonique de formule (I) et de 1 à 20 % en moles et de préférence de 1 à 15 % en moles de motif de formule (II). Dans la formule (II), la somme de n + p peut aller de 0 à 30, de préférence de 0 à 25 et mieux de 0 à 20. Les polymères particulièrement préférés sont obtenus à partir de motifs AMPS de formule (I) et de motifs de formule (II) où p = 0 ; R₁ est le radical méthyle (CH₃) ; n est un nombre entier allant de 8 à 25 et R₂ est un radical alkyle en C₁₂-C₁₅.

Les polymères pour lesquels X⁺ dans la formule (I) désigne le sodium ou l'ammonium sont plus particulièrement préférés.

Les polymères amphiphiles utilisés selon l'invention peuvent être obtenus selon les procédés classiques de polymérisation radicalaire en présence d'un ou plusieurs initiateurs tels que par exemple, l'azobisisobutyronitrile (AIBN), l'azobisdiméthylvaléronitrile, le chlorhydrate de 2,2-azobis-[2-amidinopropane] (ABAH=2,2-AzoBis-[2-Amidinopropane] Hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂O₂ éventuellement en présence de réducteurs.

Les polymères peuvent être notamment obtenus par polymérisation radicalaire en milieu tert-butanol dans lequel ils précipitent. En utilisant la polymérisation par précipitation dans le tert-butanol, il est possible d'obtenir une distribution de la taille des particules du polymère particulièrement favorable pour ses utilisations.

La réaction de polymérisation peut être conduite à une température comprise entre 0 et 150°C, de préférence entre 10 et 100°C, soit à pression atmosphérique, soit sous pression réduite. Elle peut aussi être réalisée sous atmosphère inerte, et de préférence sous azote.

Selon ce procédé, on peut obtenir le polymère préparé à partir de l'acide 2-acrylamido-2-méthylpropane-sulfonique (AMPS) ou l'un de ses sels de sodium ou d'ammonium, avec un ester de l'acide acrylique ou de l'acide méthacrylique et
- d'un alcool oxo en C₁₁ oxyéthyléné par 8 moles d'oxyde d'éthylène (GENAPOL® UD-080 de la société HOECHST/CLARIANT),
- d'un alcool oxo en C₁₁ oxyéthyléné par 7 moles d'oxyde d'éthylène (GENAPOL® UD-070 de la société HOECHST/CLARIANT),
- d'un alcool en C₁₂-C₁₄ oxyéthyléné par 7 moles d'oxyde d'éthylène (GENAPOL® LA-070 de la société HOECHST/CLARIANT),
- d'un alcool en C₁₂-C₁₄ oxyéthyléné par 9 moles d'oxyde d'éthylène (GENAPOL® LA-090 de la société HOECHST/CLARIANT),
- d'un alcool en C₁₂-C₁₄ oxyéthyléné par 11 moles d'oxyde d'éthylène (GENAPOL® LA-110 de la société HOECHST/CLARIANT),
- d'un alcool en C₁₂-C₁₅ oxyéthyléné par 23 moles d'oxyde d'éthylène.

Selon un mode préféré de réalisation de l'invention, le polymère amphiphile utilisé est de préférence un copolymère d'AMPS et de méthacrylate d'alcool en C₁₂-C₁₄ ou C₁₂-C₁₅ comportant 7 ou 23 groupes oxyéthylénés, obtenu à partir d'acide méthacrylique ou d'un sel d'acide méthacrylique et de Genapol LA-070 ou d'un alcool en C₁₂-C₁₅ oxyéthyléné par 23 moles d'oxyde d'éthylène.

De manière encore plus préférés, les polymères amphiphiles utilisés sont les copolymères d'AMPS et de méthacrylate d'alcool ayant les caractéristiques suivantes :

| Nom des chaînes pendantes | R₃ | n | Proportion molaire de monomère de formule (II) |
|---|---|---|---|
| Genapol LA-070 | Chaîne alkyle linéaire en C₁₂₋₁₄ | 7 | 8,5 % |
| Genapol LA-070 | Chaîne alkyle linéaire en C₁₂₋₁₄ | 7 | 18,8 % |
| Alcool en C₁₂-C₁₅ oxyéthyléné par 23 moles d'oxyde d'éthylène | Chaîne alkyle ramifiée en C₁₂₋₁₅ | 23 | 10,5 % |

La quantité (en matière active) de polymère(s) amphiphile(s) dans la composition de l'invention peut aller par exemple, en matière active, de 0,01 à 10 % en poids, de préférence de 0,05 à 10 % en poids, mieux de 0,05 à 5 % en poids et encore mieux de 0,25 à 3 % en poids par rapport au poids total de la composition.

### Emulsionnants lipophiles

La composition selon l'invention contient un ou plusieurs émulsionnants lipophiles. Ces émulsionnants sont caractérisés par un HLB (Hydrophilic Lipophilic Balance) inférieur ou égal à 12. Ils peuvent être choisis parmi les émulsionnants solubles ou dispersibles dans les corps gras que sont alcanes, les esters, les éthers, les triglycérides et/ou les huiles silicones. Les éléments selon l'invention sont choisis parmi les esters ou éthers de polyol ; les alcools gras ; les esters ou éthers comportant un motif sucre ; les émulsionnants siliconés ; et leurs mélanges.

Parmi les esters ou éthers de polyols, on peut citer notamment les esters de glycérol, les esters de polyéthylène glycol, les esters de sorbitan, et leurs mélanges, et par exemple le mono-isostéarate de glycéryle, tel que le produit commercialisé sous la dénomination Peceol Isostearique par la société Gattefosse, l'isostéarate de PEG-8 tel que le produit commercialisé sous la dénomination Prisorine 3644 par la société Uniqema, l'isostéarate de sorbitan tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Comme alcools gras, on peut citer les alcools gras ayant de 8 à 26 atomes de carbone, comme l'alcool cétylique, l'alcool stéarylique et leur mélange (alcool cétéarylique), l'octyl dodécanol, le 2-butyloctanol, le 2-hexyldécanol, le 2-undécylpentadécanol, l'alcool oléique et leurs mélanges.

Comme esters de sucre, on peut citer par exemple l'isostéarate de méthylglucose.

Comme émulsionnants siliconés, on peut citer par exemple les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination ABIL EM 90 par la société Goldschmidt, ou le mélange polyglycéryl-4 isostéarate/cétyl diméthicone copolyol/hexyllaurate vendu sous la dénomination ABIL WE 09 par la société Goldschmidt.

Selon un mode préféré de réalisation de l'invention, l'émulsionnant lipophile est choisi parmi les esters de glycéryle, les esters de polyéthylène glycol, les alcools gras, et leurs mélanges.

La quantité (en matière active) d'émulsionnant lipophile peut aller par exemple de 0,01 à 10 % en poids, de préférence de 0,05 à 5 % en poids et mieux de 0,1 à 2,5 % en poids par rapport au poids total de la composition.

L'émulsionnant lipophile est généralement introduit dans la phase huileuse de l'émulsion.

### Phase aqueuse

Avantageusement, le polymère amphiphile est introduit dans la phase aqueuse de l'émulsion. En outre, la phase aqueuse contient de l'eau et éventuellement un ou plusieurs composés miscibles à l'eau ou au moins en partie miscibles à l'eau, comme les polyols ; les mono-alcools inférieurs en C₂ à C₈, tels que l'éthanol et l'isopropanol ; et les cétones en C₃ à C₄ liquides à température ambiante. Par "température ambiante", il faut comprendre une température d'environ 25°C, à pression atmosphérique normale (760 mm de Hg).

Par "polyol", il faut comprendre toute molécule organique comportant au moins deux groupements hydroxyle libres. Comme polyols, on peut citer par exemple les glycols comme le butylène glycol, le propylène glycol, l'isoprène glycol, le glycérol et les polyéthylène glycols comme le PEG-8, le sorbitol, les sucres comme le glucose.

La phase aqueuse peut comprendre aussi tout additif habituel hydrosoluble ou hydrodispersible comme indiqué ci-après.

La phase aqueuse peut représenter de 10 à 99 % en poids, de préférence de 20 à 95 % en poids, mieux de 30 à 90 % en poids, et encore mieux de 40 à 85 % en poids par rapport au poids total de la composition.

Le ou les composés miscibles à l'eau, tels que polyols et alcools inférieurs, peuvent être présents en une quantité allant de 0 à 30 % du poids total de la composition notamment de 0,1 à 30 % et mieux en une quantité allant de 1 à 20%.

### Phase huileuse

La nature de la phase huileuse de l'émulsion selon l'invention n'est pas critique. La phase huileuse est une phase grasse contenant au moins un corps gras choisi parmi les huiles liquides à température ambiante (20-25°C), volatiles ou non, les gommes et les corps gras pâteux, d'origine animale, végétale, minérale ou synthétique, et leurs mélanges. Ces corps gras sont physiologiquement acceptables.

La phase huileuse comprend généralement l'émulsionnant lipophile et elle peut comprendre aussi tout additif habituel liposoluble ou lipodispersible comme indiqué ci-après.

La phase huileuse contient au moins une huile, plus particulièrement au moins une huile cosmétique. On entend par "huile" un corps gras liquide à la température ambiante (25°C).

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple, les huiles de tournesol, de maïs, de soja, de courge, de pépins de raisin, de sésame, de noisette, d'abricot, de macadamia, d'arara, de coriandre, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle ;
- les hydrocarbures linéaires ou ramifiés, d'origine minérale ou synthétique, tels que les huiles de paraffine, volatiles ou non, et leurs dérivés, la vaseline, les polydécènes, l'isohexadecane, l'isododecane, le polyisobutène hydrogéné tel que l'huile de Parléam® ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les huiles de silicone volatiles, en particulier cyclopolydiméthylsiloxanes (cyclométhicones) telles que le cyclohexadiméthylsiloxane et le cyclopentadiméthylsiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

Selon un mode préféré de réalisation, la composition de l'invention comporte au moins une huile choisie parmi les huiles de silicone, les hydrocarbures linéaires ou ramifiés, les éthers et les esters de synthèse, et leurs mélanges, et notamment choisie parmi les huiles de silicone volatiles et les hydrocarbures ramifiés comme l'huile de Parléam®, et leurs mélanges.

Les autres corps gras pouvant être présents dans la phase huileuse sont par exemple les acides gras comportant de 8 à 30 atomes de carbone, comme l'acide stéarique, l'acide laurique, l'acide palmitique ; les cires ; les gommes telles que les gommes de silicone (diméthiconol) ; les résines de silicone telles que la trifluorométhyl-C1-4-alkyldimethicone et la Trifluoropropyldimethicone, et les élastomères de silicone comme les produits commercialisés sous les dénominations "KSG" par la société Shin-Etsu, sous la dénomination "Trefil" par la société Dow Coming ou sous les dénominations "Gransil" par la société Grant Industries, et leurs mélanges.

Ces corps gras peuvent être choisis de manière variée par l'homme du métier afin de préparer une composition ayant les propriétés, par exemple de consistance ou de texture, souhaitées.

La quantité de phase huileuse dans la composition de l'invention peut aller par exemple de 1 à 90 % en poids, de préférence de 5 à 80 % en poids, mieux de 10 à 70 % en poids et encore mieux de 15 à 60 % en poids par rapport au poids total de la composition.

### Additifs

De façon connue, la composition pour application topique de l'invention peut contenir également un ou plusieurs des adjuvants habituels dans le domaine cosmétique ou dermatologique. Comme adjuvants, on peut citer par les gélifiants, les actifs, les conservateurs, les antioxydants, les parfums, les solvants, les sels, les charges, les filtres solaires (= filtres U.V.), les matières colorantes, les agents basiques (triéthanolamine, diéthanolamine, hydroxyde de sodium) ou acides (acide citrique), et encore les vésicules lipidiques ou tout autre type de vecteur (nanocapsules, microcapsules, etc...), les tensioactifs hydrophiles, et leurs mélanges. Ces adjuvants sont utilisés dans les proportions habituelles dans le domaine cosmétique, et par exemple de 0,01 à 30 % du poids total de la composition, et ils sont, selon leur nature, introduits dans la phase aqueuse de la composition ou dans la phase huileuse, ou encore dans des vésicules ou tout autre type de vecteur. Ces adjuvants ainsi que leurs concentrations doivent être tels qu'ils ne modifient pas la propriété recherchée pour l'émulsion de l'invention.

Selon la viscosité désirée pour la composition selon l'invention, on peut y incorporer un ou plusieurs gélifiants, hydrophiles ou lipophiles. Comme gélifiants hydrophiles, on peut citer par exemple les polymères carboxyvinyliques modifiés ou non, tels que les produits commercialisés sous les dénominations Carbopol (nom INCI : carbomer) et Pemulen (nom INCI : Acrylates/C10-30 akyl acrylate crosspolymer) par la société Noveon ; les polyacrylamides ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination « Hostacerin AMPS » (nom INCI : ammonium polyacryldimethyltauramide) ; les copolymères anioniques réticulés d'acrylamide et d'AMPS, se présentant sous la forme d'une émulsion E/H, tels ceux commercialisés sous le nom de SEPIGEL 305 (nom C.T.F.A. : Polyacrylamide / C13-14 Isoparaffin / Laureth-7) et sous le nom de SIMULGEL 600 (nom C.T.F.A. : Acrylamide / Sodium acryloyldimethyltaurate copolymer / Isohexadecane / Polysorbate 80) par la société SEPPIC ; les biopolymères polysaccharidiques comme la gomme de guar, les alginates, les celluloses modifiées ou non ; et leurs mélanges. Quand ils sont présents, ces gélifiants doivent être introduits en quantité telle qu'ils ne modifient pas les propriétés de la composition selon l'invention. Comme gélifiants lipophiles, on peut citer notamment les argiles modifiées telles que le silicate de magnésium modifié (bentone gel VS38 de RHEOX), l'hectorite modifiée par le chlorure de distéaryl diméthyl ammonium (nom CTFA : Disteardimonium hectorite) commercialisé sous la dénomination « bentone 38 CE » par la société RHEOX.

Comme charges qui peuvent être utilisées dans la composition de l'invention, on peut citer par exemple, les pigments tels que les oxydes de titane, de zinc ou de fer et les pigments organiques ; le kaolin ; la silice ; le talc ; le nitrure de bore ; les poudres sphériques organiques, les fibres ; et leurs mélanges. Comme poudres sphériques organiques, on peut citer par exemple les poudres de polyamide et notamment les poudres de Nylon® telles que Nylon-1 ou Polyamide 12, vendues sous les dénominations ORGASOL par la société Atochem ; les poudres de polyéthylène ; le Téflon® ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch. Comme fibres, on peut citer par exemple les fibres de polyamide, telles que notamment les fibres de Nylon 6 (ou Polyamide 6) (nom INCI : Nylon 6), de Nylon 6,6 (ou Polyamide 66) (Nom INCI : Nylon 66) ou telles que les fibres de poly-p-phénylène téréphtamide ; et leurs mélanges. Ces charges peuvent être présentes dans des quantités allant de 0 à 20 % en poids et de préférence de 0,5 à 10 % en poids par rapport au poids total de la composition.

Comme actifs utilisables dans la composition de l'invention, on peut citer par exemple, les enzymes (par exemple lactoperoxydase, lipase, protéase, phospholipase, cellulases) ; les flavonoïdes ; les agents hydratants tels que les hydrolysats de protéines ; le hyaluronate de sodium ; les polyols comme la glycérine, les glycols comme les polyéthylène glycols, et les dérivés de sucre ; les anti-inflammatoires ; les oligomères procyannidoliques ; les vitamines comme la vitamine A (rétinol), la vitamine E (tocophérol), la vitamine K, la vitamine C (acide ascorbique), la vitamine B5 (panthénol), la vitamine B3 ou PP (niacinamide), les dérivés de ces vitamines (notamment esters) et leurs mélanges ; les agents kératolytiques et/ou desquamants tels que l'acide salicylique et ses dérivés, les alpha-hydroxyacides comme l'acide lactique et l'acide glycolique et leurs dérivés, l'acide ascorbique et ses dérivés ; l'urée ; la caféine ; les dépigmentants tels que l'acide kojique, l'hydroquinone et l'acide caféique ; l'acide salicylique et ses dérivés ; les rétinoïdes tels que les caroténoïdes et les dérivés de vitamine A ; l'hydrocortisone ; la mélatonine ; les extraits d'algues, de champignons, de végétaux, de levures, de bactéries ; les stéroïdes ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les agents tenseurs tels que les polymères synthétiques, les protéines végétales, les polysaccharides d'origine végétale sous forme ou non de microgels, les amidons, les dispersions de cires, les silicates mixtes et les particules colloïdales de charges inorganiques ; les céramides ; les agents anti-inflammatoires ; les agents apaisants ; les agents matifiants ; les agents anti-chute et/ou repousse des cheveux ; les agents antirides ; les huiles essentielles ; et leurs mélanges ; et tout actif approprié pour le but final de la composition.

Comme exemples de stéroïdes, on peut citer la déhydroépiandrostérone (ou DHEA), ainsi que (1) ses précurseurs et dérivés biologiques, en particulier les sels et esters de DHEA, tels que le sulfate et le salicylate de DHEA, la 7-hydroxy DHEA, la 7-céto DHEA, les esters de 7-hydroxy et 7-céto DHEA, notamment la 3-beta-acétoxy-7-oxo DHEA, et (2) ses précurseurs et dérivés chimiques, en particulier les sapogénines telles que la diosgénine ou l'hécogénine, et/ou leurs dérivés tels que l'acétate d'hécogénine, et/ou les extraits naturels en contenant et notamment les extraits de Dioscorées, tels que l'igname sauvage (Wild Yam).

Les filtres U.V. peuvent être organiques ou minéraux (ou filtres U.V. physiques). Ils peuvent être présents en une quantité en matière active allant de 0,01 à 20 % en poids de matière active, de préférence de 0,1 à 15 % en poids, et mieux 0,2 à 10 % en poids par rapport au poids total de la composition.

Comme exemples de filtres organiques, actifs dans l'UV-A et/ou l'UV-B, pouvant être ajoutés dans la composition de l'invention, on peut citer par exemple, les dérivés à fonction sulfonique tels que les dérivés sulfonés ou sulfonatés du benzylidène camphre, de la benzophénone ou du phénylbenzimidazole, plus particulièrement les dérivés du benzylidène camphre, comme l'acide benzène 1,4 [di(3-méthylidène-campho 10-sulfonique)], (nom INCI : Terephthalylidene Dicamphor Sulfonic Acid) fabriqué sous le nom MEXORYL SX par la société CHIMEX. l'acide 4'-sulfo 3-benzylidènecamphre (nom INCI : Benzylidene Camphor Sulfonic Acid), fabriqué sous le nom MEXORYL SL par la société CHIMEX, l'acide 2-[4-(camphométhylidène) phényl] benzimidazole-5-sulfonique, l'acide phénylbenzimidazole sulfonique (nom INCI : Phenylbenzimidazole Sulfonic Acid), commercialisé sous le nom EUSOLEX 232 par la société MERCK ; les dérivés de l'acide para-aminobenzoique ; les dérivés salicyliques tels que le salicylate d'éthyl hexyle vendu sous le nom commercial NEO HELIOPAN OS par Haarmann et Reimer ; les dérivés du dibenzoylméthane tels que le Butyl Methoxydibenzoylmethane vendu notamment sous le nom commercial PARSOL 1789 par Hoffmann La Roche ; les dérivés cinnamiques tels que l'éthylhexyl Methoxycinnamate vendu notamment sous le nom commercial PARSOL MCX par Hoffmann La Roche ; les dérivés de β,β'-diphénylacrylate tels que l'octocrylene (α-cyano-β,β-diphénylacrylate de 2-éthylhexyle) vendu sous le nom commercial UVINUL N539 par la société BASF ; les dérivés de la benzophénone tels que la Benzophenone-1 vendu sous le nom commercial UVINUL 400 par BASF, la Benzophenone-2 vendu sous le nom commercial UVINUL D50 par BASF, la Benzophenone-3 ou Oxybenzone, vendu sous le nom commercial UVINUL M40 par BASF, la Benzophenone-4 vendu sous le nom commercial UVINUL MS40 par BASF ; les Dérivés du benzylidène camphre tels que le 4-Methylbenzylidene camphre vendu sous le nom commercial EUSOLEX 6300 par MERCK ; les dérivés du phenyl benzimidazole tels que le Benzimidazilate vendu sous le nom commercial NEO HELIOPAN AP par Haarmann et Reimer ; les dérivés de la triazine tels que l'Anisotriazine vendu sous le nom commercial TINOSORB S par CIBA GEIGY et l'éthylhexyl triazone vendu notamment sous le nom commercial UVINUL T150 par BASF ; les dérivés du phenyl benzotriazole tels que Drometrizole Trisiloxane vendu sous le nom commercial SILATRIZOLE par Rhodia Chimie et le Méthylène bis-Benzotriazolyl Tetramethylbutylphénol, vendu sous forme solide sous le nom commercial MIXXIM BB/100 par FAIRMOUNT CHEMICAL ou sous forme micronisé en dispersion aqueuse sous le nom commercial TINOSORB M par CIBA SPECIALTY CHEMICALS ; les dérivés anthraniliques tels que le Menthyl anthranilate vendu sous le nom commercial NEO HELIOPAN MA par Haarmann et Reimer ; les dérivés d'imidazolines ; les dérivés du benzalmalonate ; et leurs mélanges.

Comme filtres physiques pouvant être ajoutés dans la composition de l'invention, on peut citer par exemple les pigments et nanopigments d'oxydes métalliques, enrobés ou non enrobés, notamment les oxydes de titane, de fer, de zirconium, de zinc ou de cérium, et leurs mélanges, ces oxydes pouvant être sous forme de micro- ou nanoparticules (nanopigments), éventuellement enrobées.

Les compositions de l'invention sont préparées selon les procédés habituels de préparation des émulsions H/E. Le copolymère amphiphile d'AMPS est solubilisé sous agitation dans la phase aqueuse, de préférence à température ambiante (25°C), et l'émulsion est préparée par introduction de la phase huileuse dans la phase aqueuse sous agitation.

Les compositions selon l'invention peuvent se présenter par exemple sous toutes les formes galéniques des émulsions H/E, par exemple sous forme de sérum, de lait ou de crème, et elles sont préparées selon les méthodes usuelles. Les compositions, objets de l'invention, sont destinées à une application topique et peuvent constituer notamment une composition dermatologique ou cosmétique, par exemple destinée au soin, au traitement, au nettoyage et au maquillage des matières kératiniques et notamment de la peau, des lèvres, des cheveux, des cils et des ongles des êtres humains.

Selon un mode préféré de réalisation de l'invention, la composition constitue une composition cosmétique et est destinée à une application topique sur la peau.

Aussi, l'invention a encore pour objet l'utilisation cosmétique d'une composition cosmétique telle que définie ci-dessus pour le soin, le démaquillage et/ou le nettoyage de la peau, des lèvres et/ou des cheveux.

L'invention a enfin pour objet un procédé de traitement cosmétique de la peau, y compris du cuir chevelu, des cheveux, et/ou des lèvres, caractérisé par le fait qu'on applique sur la peau, les cheveux et/ou les lèvres, une composition cosmétique telle que définie ci-dessus.

Les exemples qui suivent permettront de mieux comprendre l'invention, sans toutefois présenter un caractère limitatif. Les quantités indiquées sont en % en poids, sauf mention contraire.

### I. Exemples selon l'invention

### Exemple 1 selon l'invention : Lait

| *Phase huileuse :* | |
|---|---|
| Cyclohexadimethylsiloxane | 6 % |
| Huile de Parléam® | 9 % |
| Isostéarate de glycéryle (Peceol Isostearique de Gattefosse) | 0,5 % |

| *Phase aqueuse :* | |
|---|---|
| Copolymère d'AMPS et de méthacrylate de Genapol LA-070 (avec 8,5 % en mole de monomère de formule II) | 1 % |
| Triéthanolamine à 10 % en solution aqueuse | 0,06 % |
| Conservateurs | 1 % |
| Eau | 82,44 % |

Mode opératoire : Le polymère à base d'AMPS est solubilisé dans la phase aqueuse pendant 2 heures à la température ambiante à l'aide d'une agitation mécanique. La phase huileuse est alors introduite dans la phase aqueuse sous agitation à l'aide d'un homogénéisateur de type Moritz à la vitesse de 4000 RPM pendant 20 minutes, à 25°C.

La composition obtenue se présente sous la forme d'un lait fluide et stable, de pH 6,6, ayant une viscosité de 1,7 poises (170 mPa.s), la viscosité du lait étant mesurée au Rhéomat 180 à 25°C à un gradient de cisaillement de 200 s⁻¹ au mobile 2.

### Exemple 2 selon l'invention : Lait

| *Phase huileuse :* | |
|---|---|
| Cyclohexadimethylsiloxane | 6 % |
| Huile de Parléam® | 9 % |
| Isostéarate de PEG-8 (Prisorine 3644 de la société Uniqema) | 0,5 % |

| *Phase aqueuse :* | |
|---|---|
| Copolymère d'AMPS et de méthacrylate de Genapol LA-070 (avec 8,5 % en mole de monomère de formule II) | 1 % |
| Triéthanolamine à 10 % en solution aqueuse | 0,06 % |
| Conservateurs | 1 % |
| Eau | 82,44 % |

Le mode opératoire est identique à celui de l'exemple 1.

La composition obtenue se présente sous la forme d'un lait fluide et stable, de pH 6,6, ayant une viscosité de 3,3 poises (330 mPa.s), la viscosité du lait étant mesurée au Rhéomat 180 à 25°C à un gradient de cisaillement de 200 s⁻¹ au mobile 2.

### Exemple 3 selon l'invention : Lait

| *Phase huileuse :* | |
|---|---|
| Cyclohexadimethylsiloxane | 6 % |
| Huile de Parléam® | 9 % |
| Isostéarate de glycéryle (Peceol Isostearique de Gattefosse) | 0,5 % |

| *Phase aqueuse ;* | |
|---|---|
| Copolymère d'AMPS et de méthacrylate de Genapol LA-070 (avec 18,8 % en mole de monomère de formule II) | 1 % |
| Triéthanolamine à 10 % en solution aqueuse | 0,06 % |
| Conservateurs | 1 % |
| Eau | 82,44 % |

Le mode opératoire est identique à celui de l'exemple 1.

La composition obtenue se présente sous la forme d'un lait fluide et stable.

### Exemple 4 selon l'invention : Crème

### Phase huileuse :

- Cyclopentadiméthylsiloxane 16 %
- Huile de Parléam® 12,8 %
- Fraction liquide de beurre de karité 2 %
- Acétate de tocophérol 0,2 %
- Palmitate d'octyle 3 %
- Alcool cétylique 3 %
- Alcool Stéarylique 3 %
- Propyl paraben 0,1 %

### Phase aqueuse :

- Copolymère d'AMPS et de méthacrylate de Genapol LA-070
   (8,5% en mole de monomère de formule II) 0,8 %
- Triéthanol amine 0,011 %
- Glycérine 5 %
- Conservateurs 0,5 %
- Eau qsp 100 %

Mode opératoire : Le polymère à base d'AMPS est solubilisé dans la phase aqueuse pendant 2 heures à la température ambiante à l'aide d'une agitation mécanique. La phase huileuse préalablement chauffée à 70°C est alors introduite dans la phase aqueuse également chauffée à 70°C, sous agitation à l'aide d'un homogénéisateur de type Moritz à la vitesse de 4000 RPM pendant 20 minutes. L'agitation est maintenue jusqu'au refroidissement de l'émulsion à 25°C.

La formule se présente sous la forme d'une crème (pH-6,9). Sa viscosité mesurée au Rhéomat 180 à 25°C à un gradient de cisaillement de 200s⁻¹ au mobile 3 est de 1,31 Pa.s. L'émulsion a été soumise à un test de vieillissement accéléré par centrifugation (Optima TLX Ultracentrifuge) à une vitesse de 25 000 RPM pendant 5 minutes. A l'issue de ce test, l'émulsion est restée stable.

### II. Exemples comparatifs

### Exemple 1 comparatif : lait

| *Phase huileuse :* | |
|---|---|
| Cyclohexadimethylsiloxane | 6 % |
| Huile de Parléam® | 9 % |

| *Phase* aqueuse : | |
|---|---|
| Copolymère d'AMPS et de méthacrylate de Genapol LA-070 (avec 8,5 % en mole de monomère de formule II) | 1 % |
| Triéthanolamine à 10 % en solution aqueuse | 0,06 % |
| Conservateurs | 1 % |
| Eau | 82,94 % |

Le mode opératoire est identique à celui de l'exemple 1.

La composition obtenue est un lait fluide instable, car il se produit un crémage, c'est-à-dire une remontée d'huile à la partie supérieure de l'émulsion, ce qui se traduit par une séparation de phase avec une phase blanche en haut du flacon et une phase limpide aqueuse en bas du flacon.

Cet exemple comparatif montre que la présence de tensioactif lipophile est indispensable pour obtenir une composition fluide stable.

### Exemple 2 comparatif : lait

| *Phase huileuse :* | |
|---|---|
| Cyclohexadimethylsiloxane | 6 % |
| Huile de Parléam® | 9 % |
| Sel disodique de l'acide N-stearoylglutamique (Acylglutamate HS21 de la société Ajinomoto) | 0,5 % |

| *Phase* aqueuse : | |
|---|---|
| Copolymère d'AMPS et de méthacrylate de Genapol LA-070 (avec 8,5 % en mole de monomère de formule II) | 1 % |
| Triéthanolamine à 10 % en solution aqueuse | 0,06 % |
| Conservateurs | 1 % |
| Eau | 82,44 % |

Le mode opératoire est identique à celui de l'exemple 1.

La composition obtenue est un lait fluide instable : il se produit un crémage.

L'exemple comparatif 1 montre qu'il est indispensable d'avoir un émulsionnant lipophile, et l'exemple comparatif 2 montre qu'un émulsionnant hydrophile (HLB > 12) tel que l'Acylglutamate HS21 ne permet pas de stabiliser une émulsion H/E, contenant le polymère d'AMPS modifié correspondant à la formule revendiquée.

### Exemple comparatif 3 : Crème

### Phase huileuse:

- Cyclopentadiméthylsiloxane 16 %
- Huile de Parléam® 18,8 %
- Fraction liquide de beurre de karité 2 %
- Acétate de tocophérol 0,2 %
- Palmitate d'octyle 3 %
- Propyl paraben 0,1 %

### Phase aqueuse :

- Copolymère d'AMPS et de méthacrylate de Genapol LA-070 (8,5% en mole de monomère de formule II) 0,8 %
- Triéthanol amine 0,011 %
- Glycérine 5 %
- Conservateurs 0,5 %
- Eau qsp 100 %

Le mode opératoire est identique à celui de l'exemple 1.

La formule se présente sous la forme d'une crème (pH~6,8). Sa viscosité mesurée au Rhéomat 180 à 25°C à un gradient de cisaillement de 200 s⁻¹ au mobile 3 est de 0,65 Pa.s. L'émulsion a été soumise à un test de vieillissement accéléré par centrifugation (Optima TLX Ultracentrifuge) à une vitesse de 25 000 RPM pendant 5 minutes. A l'issue de ce test, l'émulsion s'est révélée instable (observation d'un déphasage macroscopique : crémage). Cet exemple montre donc l'importance de la présence de l'émulsionnant lipophile.

## Revendications

1. Composition pour application topique, sous forme d'émulsion huile-dans-eau comprenant une phase huileuse dispersée dans une phase aqueuse, **caractérisée en ce qu'**elle contient au moins un émulsionnant lipophile de HLB inférieur ou égal à 12 choisi parmi les esters ou éthers de polyol ; les alcools gras ; les esters ou éthers comportant un motif sucre ; les émulsionnants siliconés ; et leurs mélanges et au moins un polymère amphiphile comprenant de :
(A) 80 à 99 % en moles de motif acide 2-acrylamido 2-méthylpropane sulfonique de formule (I) : 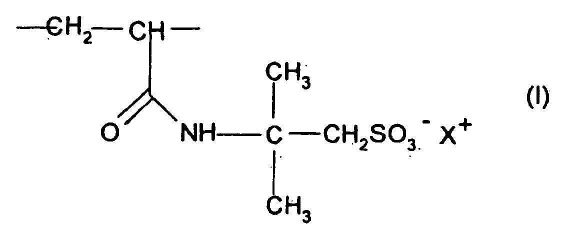 dans laquelle X⁺ est un proton, un cation de métal alcalin, un cation alcalino-terreux ou l'ion ammonium ;
et (B) 1 à 20 % en moles de motif de formule (II) suivante : 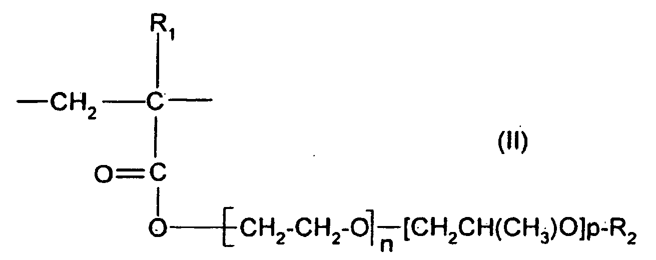
dans laquelle n et p, indépendamment l'un de l'autre, désignent un nombre entier et varie de 0 à 30, sous réserve que n + p soit inférieur ou égal à 30 ; R₁ désigne un atome d'hydrogène, un radical alkyle linéaire ou ramifié comportant de 1 à 6 atomes de carbone, et R₂ désigne un radical alkyle linéaire ou ramifié comportant de 6 à 15 atomes de carbone.

2. Composition selon la revendication 1, **caractérisée en ce que** dans la formule (II), p = 0 ; R₁ est le radical méthyle ; n est un nombre entier allant de 7 à 25 et R₂ est un radical alkyle en C₁₂-C₁₅.

3. Composition selon la revendication 1 ou 2, **caractérisée en ce que** le polymère amphiphile comprend de :
(A) 85 à 99 % en moles de motif acide 2-acrylamido 2-méthylpropane sulfonique de formule (I) et (B) 1 à 15 % en moles de motif de formule (II).

4. Composition selon l'une quelconque des revendications précédentes, **caractérisée en ce que** le polymère amphiphile est un copolymère d'acide 2-acrylamido 2-methylpropane sulfonique et de méthacrylate d'alcool en C₁₂-C₁₄ ou C₁₂-C₁₅ comportant 7 ou 23 groupes oxyéthylénés.

5. Composition selon l'une quelconque des revendications précédentes, **caractérisée en ce que** la quantité de polymère va de 0,01 à 10 % en poids par rapport au poids total de la composition.

6. Composition selon l'une quelconque des revendications précédentes, **caractérisée en ce que** l'émulsionnant lipophile est choisi parmi les esters de glycéryle, les esters de polyéthylène glycol, les alcools gras, et leurs mélanges.

7. Composition selon l'une quelconque des revendications précédentes, **caractérisée en ce que** la quantité d'émulsionnant lipophile va de 0,01 à 10 % en poids par rapport au poids total de la composition.

8. Composition selon l'une quelconque des revendications précédentes, **caractérisée en ce qu'**elle constitue une composition cosmétique ou dermatologique.

9. Utilisation cosmétique d'une composition cosmétique selon l'une quelconque des revendications 1 à 7, pour le soin, le démaquillage et/ou le nettoyage de la peau, des lèvres et/ou des cheveux.

10. Procédé de traitement cosmétique de la peau, des cheveux, et/ou des lèvres, **caractérisé par le fait qu'**on applique sur la peau, les cheveux et/ou les lèvres, une composition cosmétique selon l'une quelconque des revendications 1 à 7.

## Claims

1. Composition for topical application, in the form of an oil-in-water emulsion comprising an oily phase dispersed in an aqueous phase, **characterized in that** it contains at least one lipophilic emulsifier having an HLB of less than or equal to 12 chosen from polyol esters or ethers; fatty alcohols; esters or esthers comprising a sugar unit; silicone emulsifier; and mixtures thereof, and at least one amphiphilic polymer comprising from:
(A) 80 mol% to 99 mol% of 2-acrylamido-2-methylpropanesulphonic acid units of formula (I): 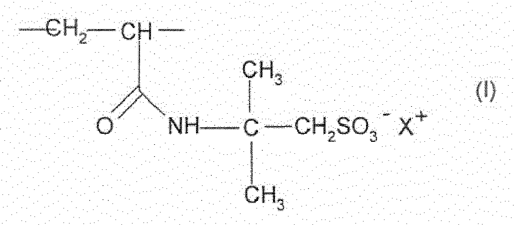 in which X⁺ is a proton, an alkali metal cation, an alkaline-earth metal cation or an ammonium iron;
and (B) 1 mol% to 20 mol% of units of formula (II) below: 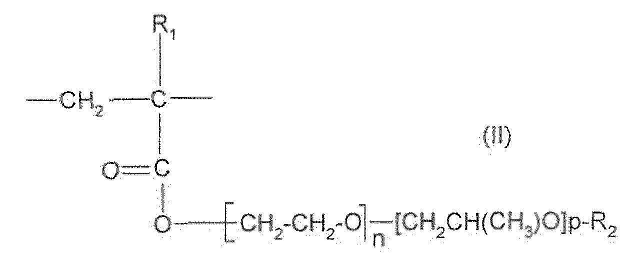
in which n and p, independently of each other, denote an integer ranging from 0 to 30, with the proviso that n + p is less than or equal to 30; R₁ denotes a hydrogen atom or a linear or branched alkyl radical containing from 1 to 6 carbon atom, and R₂ denotes a linear or branched alkyl radical containing from 6 to 15 carbon atom.

2. Composition according to Claim 1, **characterized in that**, in formula (II), p = top R₁ is a ethyl radical; n is an integer ranging from 7 to 25 and R₂ is a C₁₂-C₁₅ alkyl radical.

3. Composition according to Claim 1 or 2, **characterized in that** the amphiphilic polymer comprises from:
(A) 85 mol% to 99 mol% of 2-acrylamido-2-methylpropanesulphonic acid units of formula (I) and (B) 1 mol% to 15 mod% of units of formula (II).

4. composition according to any one of the preceding claim, **characterized in that** the amphiphilic polymer is a copolymer of 2-acrylamides-2-methylpropane-sulfonic acid and of a C₁₂-C₁₄ or C₁₂-C₁₅ alcohol methacrylate containing 7 or 23 oxyethylene groups.

5. Composition according to any one of the preceding claims, **characterized in that** the amount of polymer ranges from 0.01% to 10% by weight relative to the total weight of the composition.

6. Composition according to any one of the receding claims, **characterized in that** the lipophilic emulsifies is chosen from glyceryl esters, polyethylene glycol esters and fatty alcohol, and mixtures thereof.

7. Composition according to any one of the preceding claims, **characterized in that** the amount of lipophilic emulsifier ranges from 0.01% to 10% by weight relative to the total weight of the composition.

8. Composition according to any one of the preceding claims, **characterized in that** it is a cosmetic or dermatological composition.

9. Cosmetic use of a cosmetic composition according to any one of Claims 1 to 7, for caring for, removing makeup from and/or cleansing the skin, the lips and/or the hair.

10. Cosmetic process for treating the skin, the hair and/or the lips, **characterized in that** a cosmetic composition according to any one of Claims 1 to 7 is applied to the skin, the hair and/or the lips.

## Patentansprüche

1. Zusammensetzung zur topischen Abwendung in Form einer Öl-in-Wasser-Emulsion, die eine in einer wässrigem Phase dispergierte ölige Phase umfasst, **dadurch gekennzeichnet, dass** sie mindestens einen lipophilen Emulgator mit einem HLB-Wert kleiner gleich 12, der unter Polyolestern oder -ethern; Fettalkoholen; Estern oder Ethern mit einer Zucker-Einheit; Silikon-Emulgatoren und Mischungen davon ausgewählt ist, und mindestens ein amphiphiles Polymer, umfassend:
(A) 80 bis 99 Mol-% 2-Acrylamido-2-methylpropan-sulfonsäure-Einheit der folgenden Formel (I) : 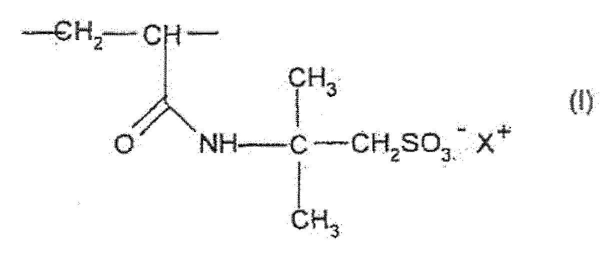 worin X⁺ für ein Proton, ein Alkalimetallkation, ein Erdalkalikation oder das Ammoniumion steht;
und (B) 1 bis 20 Mol-% der Einheit der folgenden Formel (II): 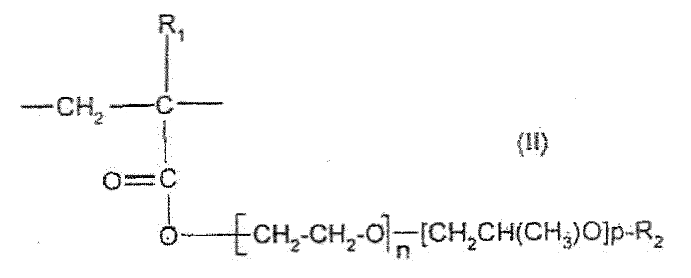
worin n und p unabhängig voneinander für eine ganze Zahl stehen und von 0 bis 30 variieren, mit der Maßgabe, dass n + p kleiner gleich 30 ist; R₁ für ein Wasserstoffatom oder einen linearen oder verzweigten Allylrest mit 1 bis 6 Kohlenstoffatomen steht und R₂ für einen linearen oder verzweigten Allylrest mit 6 bis 15 Kohlenstoffatomen steht;
umfasst.

2. Zusammensetzung nach Anspruch 1, **dadurch gekennzeichnet, dass** in der Formel (II) p = 0; R₁ für den Methylrest steht; n für eine ganze Zahl von 7 bis 25 steht und R₂ für einen C₁₂-C₁₅-Alkylrest steht.

3. Zusammensetzung nach Anspruch 1 oder 2, **dadurch gekennzeichnet, dass** das amphiphile Polymer (A) 85 bis 99 Mol-% 2-Acrylamido-2-methylpropan-sulfonsäure-Einheit der Formel (I) und (B) 1 bis 15 Mol-% der Einheit der Formel (II) umfasst.

4. Zusammensetzung nach einem der vorhergehenden Ansprüche, **dadurch gekennzeichnet, dass** es sich bei dem amphiphilen Polymer um ein Copolymer von 2-Acrylamido-2-methylpropan-sulfonsäure-Einheit und einem Methacrylat eines C₁₂-C₁₄-Alkohols oder C₁₂-C₁₅-Alkohols mit 7 bis 23 Oxyethylengruppen handelt.

5. zusammensetzung nach einem der vorhergehenden Ansprüche, **dadurch gekennzeichnet, dass** die Polymermenge 0,01 bis 10 Gew.-%, bezogen auf das Gesamtgewicht der Zusammensetzung, beträft.

6. zusammensetzung nach einem der vorhergehenden Anspruche, **dadurch gekennzeichnet, dass** der lipophile Emulgator unter Glycerylestern, Polyethylenglykolestern, Fettalkoholen und Mischungen davon ausgewählt ist.

7. Zusammensetzung nach einem der vorhergehenden Ansprüche, **dadurch gekennzeichnet, dass** die Menge an lipophilem Emulgator 0,01 bis 10 Gew.-%, bezogen auf das Gesamtgewicht der Zusammensetzung, beträgt.

8. Zusammensetzung nach einem der vorhergehenden Ansprüche, dadurch gekenntzeichnet, das es sich um eine kosmetische oder dermatologische zusammensetzung handelt.

9. Kosmetische Verwendung einer kosmetischen Zusammensetzung nach einem der Ansprüche 1 bis 7 zur Pflege, Abschminkung und/oder Reinigung der Haut, der Lippen und/oder der Haare.

10. verfahren zur kosmetischen Behandlung der Haut, der Haare und/oder der Lippen, dadurch gekennzeichnen, dass man auf die Haut, die Haare und/oder die Lippen eine kosmetische Zusammensetzung nach einem der Anspruche 1 bis 7 aufbringt.
